# EUROPÄISCHE PATENTSCHRIFT

(11) **EP 4 162 237 B1**
(45) Veröffentlichungstag und Bekanntmachung des Hinweises auf die Patenterteilung: **01.07.2026**
(21) Anmeldenummer: 21730546.5
(22) Anmeldetag: 31.05.2021
(51) Int. Cl.: G01F 23/00, G01F 23/32, A61M 16/16, F24F 6/02, A61M 16/10, A61M 16/00, A61M 16/14

(54) **SCHWIMMERGESTEUERTE VENTILANORDNUNG MIT REDUNDANT WIRKENDEN SCHWIMMERKÖRPERN**
FLOAT-CONTROLLED VALVE ARRAY HAVING REDUNDANTLY ACTING FLOAT BODIES
RÉSEAU DE VANNES COMMANDÉ PAR FLOTTEUR COMPORTANT DES CORPS FLOTTANTS À ACTION REDONDANTE

(30) Priorität: 04.06.2020 DE 102020114921
(43) Veröffentlichungstag der Anmeldung: 12.04.2023
(73) Patentinhaber: Hamilton Medical AG, 7402 Bonaduz (CH)
(72) Erfinder: DE-STEFANI, Dino, 7000 Chur (CH); SENN, Remo, 7205 Zizers (CH)
(74) Vertreter: Ruttensperger Lachnit Trossin Gomoll
(86) Internationale Anmeldenummer: PCT/EP2021/064561
(87) Internationale Veröffentlichungsnummer: WO 2021/245030

(56) Entgegenhaltungen:
- DE-A1- 19 711 192
- DE-A1- 3 830 314
- DE-C- 535 721
- DE-C- 640 283

## Beschreibung

Die vorliegende Erfindung betrifft eine schwimmergesteuerte Ventilanordnung, insbesondere zur Verwendung in Befeuchtungsvorrichtungen von Beatmungsvorrichtungen zur künstlichen Beatmung von Menschen oder Tieren. Die Erfindung betrifft außerdem eine Befeuchtungsvorrichtung mit einer solchen Ventilanordnung.

Die schwimmergesteuerte Ventilanordnung umfasst eine Ventilbaugruppe mit einem Kanal, einer von dem Kanal durchsetzten Ventilsitzformation und einer Ventilkörperformation, wobei die Ventilkörperformation relativ zur Ventilsitzformation verlagerbar ist zwischen einer Verschlussstellung, in welcher der Kanal durch körperliche Anlage der Ventilkörperformation an der Ventilsitzformation verschlossen ist, und einer Durchlassstellung, in welcher die Ventilkörperformation mit Abstand von der Ventilsitzformation angeordnet ist, so dass der Kanal durchströmbar ist. Die Ventilanordnung umfasst weiter einen ersten Schwimmerkörper mit einem ersten Auftriebsvolumenabschnitt und einen zweiten Schwimmerkörper mit einem zweiten Auftriebsvolumenabschnitt. Der erste Schwimmerkörper ist an einem ersten Gelenk schwenkbar angelenkt und der zweite Schwimmerkörper ist an einem zweiten Gelenk schwenkbar angelenkt. Folglich ist jeder Schwimmerkörper im bestimmungsgemäßen Betrieb der Ventilanordnung längs einer zur Schwerkraftwirkungsrichtung parallelen Auftriebsachse bewegbar zwischen einer Absenkstellung und einer Auftriebsstellung. Der erste und der zweite Schwimmerkörper sind dabei jeweils derart mit der Ventilkörperformation gekoppelt, dass die Ventilkörperformation sich dann in der Verschlussstellung befindet, wenn sich wenigstens einer der Schwimmerkörper in der Auftriebsstellung befindet, und sich dann in der Durchlassstellung befindet, wenn sich beide Schwimmerkörper in der Absenkstellung befinden.

Eine solche schwimmergesteuerte Ventilanordnung und eine Befeuchtungsvorrichtung mit dieser sind aus der US 5,445,143 bekannt. Der erste Schwimmerkörper dieser bekannten Ventilanordnung dient als Haupt-Schwimmerkörper, welcher maßgeblich die Stellung der Ventilkörperformation relativ zur Ventilsitzformation bestimmen soll. Der zweite Schwimmerkörper dient als Reserve-Schwimmerkörper, welcher die Aufgaben des ersten Schwimmerkörpers übernehmen soll, falls der erste Schwimmerkörper versagt. Die Füllstände und die Füllmengen, bei welchen der erste und der zweite Schwimmerkörper ihre Auftriebsstellung erreichen und dadurch jeweils für einen Verschluss des Kanals sorgen, unterscheiden betragsmäßig sich erheblich. Der Auftriebsvolumenabschnitt eines jeden Schwimmerkörpers, welcher im Zusammenwirken mit einer Flüssigkeitsmenge für die zur Verstellung des zugehörigen Schwimmerkörpers in die Auftriebsstellung notwendige Auftriebskraft sorgt oder maßgeblich sorgt, ist starr mit einem Steg verbunden. Jeder Steg ist über ein Gelenk an einem Einsatz im Füllvolumen des die bekannte Ventilanordnung aufweisenden Behälters der Befeuchtungsvorrichtung angelenkt. Wegen der Schwenkbeweglichkeit der Schwimmerkörper weist die Trajektorie ihrer Auftriebsvolumenabschnitte zwischen Absenk- und Auftriebsstellung nicht nur eine Bewegungskomponente längs der Auftriebsachse auf, sondern auch, jedoch in geringerem Umfang, orthogonal zu dieser. Die beiden Schwimmerkörper sind entweder längs der Auftriebsachse übereinander angeordnet, dann können sie unterschiedlich groß oder gleich groß sein, oder die beiden Schwimmerkörper sind orthogonal zur Auftriebsachse nebeneinander angeordnet, dann müssen Sie unterschiedlich groß sein, damit der eine als Haupt- und der andere als Reserve-Schwimmerkörper wirkt.

Eine weitere Ventilanordnung mit einem Haupt- und einem Reserve-Schwimmerkörper ist aus der EP 2 119 466 A1 bekannt. Anders als in der vorgenannten US 5,445,143 sind der erste und der zweite Schwimmerkörper hier jeweils translatorisch nur längs der Auftriebsachse zwischen der Absenkstellung und der Auftriebsstellung beweglich. Die translatorische Beweglichkeit ermöglicht eine konzentrische Anordnung der beiden Schwimmerkörper bezüglich der Auftriebsachse. Der äußere Reserve-Schwimmerkörper ist am Gehäuse der Befeuchtungsvorrichtung zur Bewegung zwischen seinen Betriebsstellungen geführt. Der innere Haupt-Schwimmerkörper ist am Reserve-Schwimmerkörper geführt. Durch ein Verkanten auch nur eines der Schwimmerkörper kann somit die gesamte Schwimmerkörperanordnung in ihrer Bewegung blockiert werden. Auch für die aus der EP 2 119 466 A1 bekannte Ventilanordnung gilt, dass sich die Füllstände und die Füllmengen in der die Ventilanordnung aufweisenden Befeuchtungsvorrichtung, welche eine Bewegung des Haupt- und des Reserve Schwimmerkörpers in die Auftriebsstellung bewirken, betragsmäßig erheblich unterscheiden.

Der Kanal der Ventilanordnung ist in der Regel ein Kanal, durch welchen Flüssigkeit in ein Füllvolumen fließen kann, in welchem die Schwimmerkörper angeordnet sind. Dadurch wird im Stand der Technik bei Erreichen einer Füllhöhe, welche durch die konstruktive Ausgestaltung und die Anordnung des Haupt-Schwimmerkörpers bestimmt ist, der Schwimmerkörper in die Auftriebsstellung bewegt, wodurch die Ventilkörperformation den Kanal verschließt. Nimmt der Füllstand im Bereich der Schwimmerkörper ab, beispielsweise durch Verdunstung und Abtransport von Wasserdampf, wird der Schwimmerkörper schwerkraftgetrieben aus seiner Auftriebsstellung zurück in Richtung Absenkstellung verlagert, wodurch die Ventilkörperformation von der Ventilsitzformation abhebt und Flüssigkeit durch den Kanal erneut in den Bereich der Schwimmerkörper nachströmen kann. Somit kann durch die Ventilanordnung die Füllmenge an Flüssigkeit im Bereich der Schwimmerkörper reguliert und auf eine maximale Füllmenge begrenzt werden, solange der Haupt-Schwimmerkörper korrekt funktioniert.

Diese Begrenzung der maximalen Füllmenge ist vor allem in Befeuchtungsvorrichtungen für Beatmungsvorrichtungen zur künstlichen Beatmung wichtig. Durch eine solche Befeuchtungsvorrichtung soll Beatmungsgas für den zu beatmeten Patienten befeuchtet werden, damit das Beatmungsgas auch bei länger dauernder Beatmung für den Patienten verträglich ist und Abschnitte im körperlichen Atmungsapparat nicht durch zu trockenes Beatmungsgas ausgetrocknet werden. Dabei ist jedoch auf jeden Fall zu vermeiden, dass Flüssigkeit vom Beatmungsgas mitgerissen wird und die Patientenlunge erreicht.

Dabei ist nicht nur ein zu hoher Füllstand eine mögliche Gefahrenquelle für einen unerwünschten Flüssigkeitstransport. Auch ein zunächst korrekter Füllstand kann die Gefahr eines Flüssigkeitstransports begründen, wenn sich die korrekte Flüssigkeitsmenge innerhalb einer Befeuchtungsvorrichtung am falschen Ort akkumuliert. Dies kann beispielsweise beim Kippen der Befeuchtungsvorrichtung und mit ihr der Ventilanordnung geschehen. Zum einen kann sich die Flüssigkeitsmenge dadurch einer Auslassöffnung nähern, was einen Abtransport von Flüssigkeit aus der Befeuchtungsvorrichtung fördern kann. Zum anderen wird durch Kippen der Ventilanordnung die Auftriebsachse als die für die Bewegung der Schwimmerkörper zwischen Absenkstellung und Auftriebsstellung relevante Bewegungsbahnkomponente von der Schwerkraftwirkungsrichtung weg geneigt. Als Folge davon kann sich der wenigstens eine Schwimmerkörper, der sich bisher korrekterweise in der Auftriebsstellung befand, aus dieser wegbewegen, sodass weitere Flüssigkeit in das Füllvolumen nachströmen kann, obwohl sich bereits die korrekte maximale Flüssigkeitsmenge in der Befeuchtungsvorrichtung befindet.

Aus der DE 640 283 C ist eine gattungsgemäße Ventilanordnung für einen Spritzvergaser von Brennkraftmaschinen an Flugzeugen oder Geländewagen bekannt. Diese Ventilanordnung soll bei Schräglage des Vergasers eine Kraftstoffzufuhr unterbrechen. Die beiden Schwimmerkörper sind mit jeweiligen Gelenken um eine koaxiale Schwenkachse schwenkbar. Bezüglich der Schwenkachse befinden sich die Gelenke im axialen Erstreckungsbereich eines jeden Schwimmerkörpers, mit welchem das jeweilige Gelenk zusammenwirkt. Daher ist der Abstand der beiden Gelenke der Schwimmerkörper größer als der Abstand der Schwimmerkörper voneinander, jedoch nicht so groß, als dass die Schwimmerkörper sich im Abstandsraum zwischen den Gelenken befänden. Durch die bekannte Anordnung bewirkt die Auftriebskraft auf jeden Schwimmerkörper nur ein Schwenkmoment um die jeweilige Schwenkachse, jedoch kein Kippmoment orthogonal zur Schwenkachse.

Aus der Druckschrift DE 197 11 192 A1 ist ein Doppelschwimmer für einen Vergaser von Brennkraftmaschinen bekannt. Der Doppelschwimmer weist zwei Schwimmerkörper auf, welche starr miteinander verbunden sind und nur gemeinsam um ein einziges gemeinsames Gelenk schwenkbar sind, welches im Abstandsraum zwischen den beiden Schwimmerkörpern angeordnet ist.

Aus der DE 38 30 314 A1 ist ein Gas- oder Luft befeuchtete bekannt, welche in einer Ausgestaltung zwei um eine koaxiale Schwenkachse schwenkbare Schwimmerkörper aufweist, welche gesondert voneinander zwischen ihrer jeweiligen Absenkstellung und Auftriebsstellung beweglich sind, wobei der eine Schwimmerkörper ein Zuflussventil mit einem Zuflusskanal und der jeweils andere Schwimmerkörper ein Abflussventil mit einem vom Zuflusskanal gesonderten Abflusskanal abhängig von ihrer Betriebsstellung öffnet oder schließt.

Die relative Anordnung der Schwimmerkörper und ihre Schwenkachse der aus den Druckschriften DE 640 283 C, DE 197 11 192 A1 und DE 38 30 314 A1 bekannten Ventilanordnungen ist im Wesentlichen identisch.

Aus der DE 535 721 C ist eine Ventilvorrichtung für einen Schnellstromverdampfer mit miteinander zur gemeinsamen Bewegung gekoppelten Schwimmerkörpern bekannt.

Es ist Aufgabe der vorliegenden Erfindung, eine wie eingangs beschrieben ausgestaltete Ventilanordnung derart weiterzubilden, dass eine Überfüllung eines durch die Ventilanordnung gesicherten Füllvolumens mit einer durch den Kanal strömenden Flüssigkeit auch bei einer Lageabweichung der Ventilanordnung von einer bestimmungsgemäßen Soll-Betriebslage verhindert werden kann.

Die vorliegende Erfindung löst diese Aufgabe an der eingangs genannten Ventilanordnung dadurch, dass - bei Betrachtung der beiden Schwimmerkörper in ihrer jeweiligen Absenkstellung als einem Bezugszustand - die jeweiligen Auftriebsvolumenabschnitte der beiden Schwimmerkörper mit einem zur Auftriebsachse orthogonalen Abstand voneinander angeordnet sind, wobei in einem Körper-Abstandsbereich zwischen beiden Auftriebsvolumenabschnitten wenigstens ein Gelenk gelegen ist oder/und wobei in einem Gelenk-Abstandsbereich zwischen beiden Gelenken wenigstens ein Auftriebsvolumenabschnitt gelegen ist.

Sofern nichts Anderes explizit ausgesagt ist, soll der Betriebszustand der Ventilanordnung, in welcher sich beide Schwimmerkörper jeweils in der Absenkstellung befinden, der Bezugszustand sein, in welchem die vorliegende Ventilanordnung beschrieben wird. Die Absenkstellung ist dabei jene Stellung, welche ein Schwimmerkörper in seinem Behälter einnimmt, wenn der Behälter frei von Flüssigkeit ist.

Die Auftriebsachse ist jene geradlinige Achse, längs welcher eine Auftriebskraft wirkt, wenn ein die Ventilanordnung tragender Behälter im bestimmungsgemäß betriebsbereiten Zustand auf einem ebenen, horizontalen, also zur Schwerkraftwirkungsrichtung orthogonalen Untergrund abgestützt ist. Im bestimmungsgemäß betriebsbereiten Zustand verläuft die Auftriebsachse parallel zur Schwerkraftwirkungsrichtung. Wird jedoch der die Ventilanordnung aufweisende Behälter bezüglich seines bestimmungsgemäß betriebsbereiten Zustands um eine zur Schwerkraftwirkungsrichtung orthogonale Kippachse um einen Kippwinkel verkippt, ist die Auftriebsachse relativ zur Schwerkraftwirkungsrichtung um den Kippwinkel geneigt.

Der oben genannte Bezugszustand ist stets ein bestimmungsgemäß betriebsbereiter Zustand, in welchen die Auftriebsachse parallel zur Schwerkraftwirkungsrichtung ist.

Wenn im Übrigen ausgesagt ist, dass die Ventilkörperformation vom Ventilsitz dann abgehoben ist, wenn sich die Schwimmerkörper in der Absenkstellung befinden, so soll dies nicht ausschließen, dass die Ventilkörperformation auch dann vom Ventilsitz abgehoben ist, wenn sich die Schwimmerkörper in einer Zwischenstellung zwischen der Absenkstellung und der Auftriebsstellung befinden. Tatsächlich wird in den meisten Fällen die Ventilkörperformation vom Ventilsitz abgehoben sein, wenn sich kein Schwimmerkörper in der Auftriebsstellung befindet.

Durch die Ausbildung von Abstandsbereichen, welche in einer allgemeinen Ausgestaltung der vorliegenden Erfindung in ihrer Erstreckung längs der Auftriebsachse unbegrenzt sein sollen, ist es möglich, die beiden Auftriebsvolumenabschnitte in unterschiedlichen, orthogonal zur Auftriebsachse nebeneinander liegenden Raum- und Flächenbereichen des Füllvolumens eines die Ventilanordnung aufnehmenden Behälters anzuordnen. In der Folge dieser Anordnung mit räumlichem Abstand voneinander können die Schwimmerkörper, die gleichsam als Sensoren zur Erfassung eines Füllstands wirken, die Füllhöhe einer in das Füllvolumen eingefüllten Flüssigkeit in unterschiedlichen, orthogonal zur Auftriebsachse mit Abstand voneinander gelegenen Bereichen des Füllvolumens erfassen. So kann wenigstens einer der Auftriebsvolumenabschnitte außermittig bezüglich des Füllvolumens angeordnet sein, sodass er im bestimmungsgemäßen betriebsbereiten Zustand die Füllhöhe über dem Grund des Füllvolumens erfassen und sodass er auch im gekippten Zustand des Behälters durch eine Akkumulation in die Auftriebsstellung verlagert werden und so den Kanal für eine Durchleitung von Flüssigkeit verschließen kann.

Die Schwimmerkörper der vorliegenden Ventilanordnung sind, anders als im Stand der Technik, nicht hierarchisch als Haupt- und Reserve-Schwimmerkörper angeordnet, sondern als gleichberechtigte Schwimmerkörper. Dies ist aufgrund der oben beschriebenen Anordnung der Auftriebsvolumenabschnitte mit räumlichen Abstand voneinander vorteilhaft, da so jeder einzelne Schwimmerkörper dann, wenn in seinem Anordnungsbereich ausreichend Flüssigkeit akkumuliert ist, in die Auftriebsstellung auftreibt und den Kanal verschließt.

"Gleichberechtigt" bedeutet hier, dass sich im bestimmungsgemäß betriebsbereiten Zustand die Flüssigkeitsmenge, bei welcher der erste Schwimmerkörper in die Auftriebsstellung verlagert wird, und die Flüssigkeitsmenge, bei welcher der zweite Schwimmerkörper in die Auftriebsstellung verlagert wird, um nicht mehr als 10 %, vorzugsweise um nicht mehr als 7,5 % bezogen auf die größere der beiden Flüssigkeitsmengen unterscheiden. Selbstverständlich sind die beiden Flüssigkeitsmengen Mengen einer identischen Flüssigkeit.

Zur Kennzeichnung eines Abschnitts eines Schwimmerkörpers als zu dem Schwimmerkörper zugehörig ist der Abschnitt in der vorliegenden Anmeldung mit derselben Ordinalzahl bezeichnet wie der Schwimmerkörper, dessen Abschnitt er bildet. Ein erster Auftriebsvolumenabschnitt ist daher beispielhaft ein Auftriebsvolumenabschnitt des ersten Schwimmerkörpers und dergleichen.

Grundsätzlich kann es ausreichen, wenn die Gelenke und die Auftriebsvolumenabschnitte längs einer mit der Auftriebsachse einen, vorzugsweise rechten, Winkel einschließenden Richtung alternierend aufeinander folgend angeordnet sind. Durch diese Anordnung kann beispielsweise ein Auftriebsvolumenabschnitt die Füllhöhe von Flüssigkeit in einem dem Zentrum eines Füllvolumens näherliegenden Zentralbereich erfassen und ein weiterer Auftriebsvolumenabschnitt in einem dem Rand des Füllvolumens näherliegenden Randbereich. Ein Verkippen der Ventilanordnung in jeder von zwei entgegengesetzten Kipprichtungen um eine Kippachse kann dann vorteilhaft zu einem erwünschten Verschließen des Kanals führen, wenn im Bezugszustand beide Gelenke in dem Körper-Abstandsbereich gelegen sind. Dies ermöglicht die Anordnung der Auftriebsvolumenabschnitte der Schwimmerkörper mit betragsmäßig großem Abstand voneinander. Die beiden Auftriebsvolumenabschnitte sind dann auf unterschiedlichen Seiten der Gelenke gelegen bzw. die Gelenke zwischen den Auftriebsvolumenabschnitten.

Für ein Erfassen der Füllhöhe und ein Verschließen des Kanals bei Überschreiten einer Grenz-Füllhöhe im bestimmungsgemäß betriebsbereiten Zustand spielt die räumliche Anordnung der Auftriebsvolumenabschnitte in Richtungen orthogonal zur Schwerkraftwirkungsrichtung bestenfalls eine untergeordnete Rolle, verglichen mit ihrer räumlichen Anordnung längs der Schwerkraftwirkungsrichtung. Denn im bestimmungsgemäß betriebsbereiten Zustand verläuft ein Flüssigkeitsspiegel einer in das Füllvolumen eines die Ventilanordnung aufnehmenden Behälters eingefüllten Flüssigkeit orthogonal zur Auftriebsachse. Ein Verschieben eines Auftriebsvolumenabschnitts orthogonal zur Auftriebsachse ändert somit an dem durch den Auftriebsvolumenabschnitt bewirkten Auftrieb nichts.

Die Kippachse einer besonders sicher durch die Schwimmerkörper zu erfassenden Verkippung verläuft in einem, vorzugsweise rechten, Winkel zur Auftriebsachse durch den Abstandsbereich, der bevorzugt ein Körper-Abstandsbereich ist.

Vorteilhafterweise ist wenigstens ein Auftriebsvolumenabschnitt derart schwenkbar angeordnet, dass eine Trajektorie seiner Verlagerung zwischen Absenkstellung und Auftriebsstellung, im Zweifel seine Schwerpunkt-Trajektorie, eine überwiegende parallel zur Auftriebsachse verlaufende Bewegungskomponente und nur eine untergeordnete zur Auftriebsachse orthogonale Bewegungskomponente aufweist. Dies kann dadurch realisiert sein, dass wenigstens ein in dem Körper-Abstandsbereich gelegenes Gelenk in einem sich längs der Auftriebsachse erstreckenden Höhenerstreckungsbereich angeordnet ist, in welchem sich im Bezugszustand auch wenigstens ein Auftriebsvolumenabschnitt, vorzugsweise beide Auftriebsvolumenabschnitte, erstrecken.

Bevorzugt erstrecken sich die Auftriebsvolumenabschnitte beider Schwimmerkörper zur Erzielung möglichst einheitlicher Auftriebskräfte bei einer im bestimmungsgemäß betriebsbereiten Zustand eingefüllten Füllmenge bezogen auf ihr Volumen zu wenigstens 60 % in einem gemeinsamen sich längs der Auftriebsachse erstreckenden Höhenerstreckungsbereich, vorzugsweise vollständig in einem gemeinsamen Höhenerstreckungsbereich. Aus den gleichen Gründen bevorzugt unterscheiden sich die Höhenabmessungen beider Auftriebsvolumenabschnitte im Bezugszustand um nicht mehr als 10 %, vorzugsweise um nicht mehr als 7,5 %, bezogen auf die größere Höhenabmessung, besonders bevorzugt sind die Höhenabmessungen beider Auftriebsvolumenabschnitte im Bezugszustand identisch.

Grundsätzlich können die beiden virtuellen Schwenkachsen, um welche der erste und der zweite Schwimmerkörper schwenkbar an ihren jeweiligen Gelenken angelenkt sind, längs der Auftriebsachse mit Abstand voneinander angeordnet sein. Vorteilhaft wenig unterschiedliche Kinematiken bei einer Verstellung zwischen der Absenkstellung und der Auftriebsstellung mit zueinander wenig unterschiedlichen Bewegungsanteilen längs der Auftriebsachse einerseits und orthogonal zu dieser andererseits können dadurch erhalten werden, dass eine erste virtuelle Schwenkachse, um welche der erste Schwimmerkörper schwenkbar am ersten Gelenk angelenkt ist, und eine zweite virtuelle Schwenkachse, um welche der zweite Schwimmerkörper schwenkbar am zweiten Gelenk angelenkt ist, in einer gemeinsamen virtuellen Erstreckungsebene gelegen sind, wobei die virtuelle Erstreckungsebene bevorzugt wenigstens einen Auftriebsvolumenabschnitt im Bezugszustand schneidet. Die beiden virtuellen Schwenkachsen sind bevorzugt zu einander parallel. Die virtuellen Schwenkachsen sind besonders bevorzugt im Verhältnis zum Abstand zu jedem Auftriebsvolumenabschnitt längs der Auftriebsachse so nahe beieinander angeordnet, dass die von beiden Schwenkachsen aufgespannte Ebene im Bezugszustand beide Auftriebsvolumenabschnitte schneidet.

Eine vorteilhaft ähnliche oder sogar gleiche Bewegung führen die beiden Schwimmerkörper zwischen ihrer Absenkstellung und ihrer Auftriebsstellung dann aus, wenn die virtuelle Erstreckungsebene orthogonal zur Auftriebsachse ausgerichtet ist. Da die Auftriebsachse in dem bestimmungsgemäßen Betriebszustand parallel zur Schwerkraftwirkungsrichtung verläuft, ist in diesem Betriebszustand ein Flüssigkeitsspiegel einer in das Füllvolumen eines die Ventilanordnung tragenden Behälters eingefüllten Flüssigkeit ebenfalls orthogonal zur Auftriebsachse.

In einer bevorzugt kompakten Ausführung kann die Ventilbaugruppe ein Ventilgehäuse umfassen, an welchem der Kanal ausgebildet ist. Der im Ventilgehäuse ausgebildete Kanal kann Teil einer längeren Leitung sein, die bis zu einem Flüssigkeitsvorrat führen kann. Jedes Gelenk umfasst einen am Schwimmerkörper angeordneten oder ausgebildeten schwimmerkörperseitigen Gelenkabschnitt und einen mit dem schwimmerkörperseitigen Gelenkabschnitt wechselwirkenden, an einem Schwenklager angeordneten oder ausgebildeten lagerseitigen Gelenkabschnitt. Der lagerseitige Gelenkabschnitt kann an einem beliebigen Abschnitt eines die Ventilanordnung tragenden Behälters ausgebildet sein. Zur Erleichterung der Anordnung der Ventilbaugruppe als vormontierte Baugruppe an oder in einem Behälter ist bevorzugt der lagerseitige Gelenkabschnitt wenigstens eines Gelenks, vorzugsweise beider Gelenke, am Ventilgehäuse ausgebildet.

Grundsätzlich kann das Ventilgehäuse aus mehreren gesondert hergestellten Bauteilen gebaut sein. Bevorzugt ist das Ventilgehäuse zur Erleichterung von Fertigung und Montage einstückig oder zweistückig, etwa durch zwei Halb- oder Teilschalen, ausgebildet. Dies ist beispielsweise bei einem spritzgegossenen Ventilgehäuse auch mit verhältnismäßig komplexer Bauteilgeometrie möglich.

Da der durch die Ventilanordnung zu verschließende oder für eine Durchströmung freizugebende Kanal im Verhältnis zum Füllvolumen des die Ventilanordnung aufnehmenden Behälters eine in der Regel sehr kleine Durchmesserabmessung aufweist, müssen die beiden Schwimmerkörper häufig in räumlich sehr nahe beieinanderliegenden Bereichen auf die Ventilkörperformation einwirken. Dies kann dadurch erleichtert werden, dass der erste Auftriebsvolumenabschnitt näher an der zweiten Schwenkachse als an der ersten Schwenkachse gelegen ist oder/und dass der zweite Auftriebsvolumenabschnitt näher an der ersten Schwenkachse als an der zweiten Schwenkachse gelegen ist.

Bevorzugt weist wenigstens ein Schwimmerkörper den Auftriebsvolumenabschnitt, den schwimmerkörperseitigen Gelenkabschnitt und einen den Auftriebsvolumenabschnitt mit dem schwimmerkörperseitigen Gelenkabschnitt verbindenden Verbindungsabschnitt auf. Denn bevorzugt ist der Auftriebsvolumenabschnitt wenigstens eines Schwimmerkörpers, vorzugsweise beider Schwimmerkörper, mit Abstand von der Schwenkachse desselben Schwimmerkörpers angeordnet, damit der Schwimmerkörper zwischen Absenkstellung und Auftriebsstellung einen für eine Verlagerung der Ventilkörperformation ausreichenden Bewegungsweg ausführt.

Ein solcher Verbindungsabschnitt kann eine Stegformation sein. Damit kann der Auftriebsvolumenabschnitt auch entfernt von dem Gelenk seines Schwimmerkörpers im Füllvolumen, etwa an einem Randbereich desselben, angeordnet sein. Stärker bevorzugt sind daher beide Schwimmerkörper in der genannten Weise ausgebildet. Die beiden Schwimmerkörper können, wie oben dargelegt, daher derart verschränkt angeordnet sein, dass deren Auftriebsvolumenabschnitte im Bezugszustand, und vorzugsweise auch dann, wenn sich die Schwimmerkörper in der Auftriebsstellung befinden, auf unterschiedlichen Seiten der vorteilhaft zwischen ihnen verlaufenden Schwenkachsen gelegen sind. Jeder Auftriebsvolumenabschnitt ist über den Verbindungsabschnitt mit seinem Gelenk verbunden, wobei die Schwenkachse des jeweils anderen Auftriebsvolumenabschnitts dem Auftriebsvolumenabschnitt näher gelegen ist als die eigene Schwenkachse. Zwischen den beiden Schwenkachsen überlappen sich daher die Verbindungsabschnitte der beiden Schwimmerkörper.

Grundsätzlich können die Schwimmerkörper über eine beliebige Konstruktion mittels Getriebe oder/und Geschiebe oder/und Gestänge mit der Ventilkörperformation gekoppelt sein, sodass eine Bewegung des Schwimmerkörpers in die Auftriebsstellung eine Bewegung der Ventilkörperformation in die Verschlussstellung bewirkt.

Eine besonders einfache, jedoch wirksame Kopplung eines jeden Schwimmerkörpers mit der Ventilkörperformation, welche eine direkte Bewegungsübertragung vom Schwimmerkörper, insbesondere von dessen Verbindungsabschnitt, zur Ventilkörperformation gestattet, kann in einem zwischen den beiden Schwenkachsen gelegenen Kopplungsbereich realisiert sein. Bevorzugt ist daher vorgesehen, dass eine Kopplung des ersten Schwimmerkörpers mit der Ventilkörperformation in einem Kopplungsbereich zwischen der ersten und der zweiten Schwenkachse gelegen ist oder/und dass eine Kopplung des zweiten Schwimmerkörpers mit der Ventilkörperformation in einem Kopplungsbereich zwischen der ersten und der zweiten Schwenkachse gelegen ist. Der genannte Kopplungsbereich erstreckt sich im Zweifel parallel zur Auftriebsachse und wird begrenzt durch zwei eine zur Auftriebsachse parallele Ebenen, von welchen jede genau eine Schwenkachse enthält.

Wenngleich grundsätzlich eine Kopplung des Schwimmerkörpers mit der Ventilkörperformation als Kopplung des Auftriebsvolumenabschnitts mit der Ventilkörperformation ausgestaltet sein kann, ist eine Kopplung des Verbindungsabschnitts des Schwimmerkörpers mit der Ventilkörperformation bevorzugt, da der Verbindungsabschnitt hinsichtlich seiner Gestalt nahezu frei konfigurierbar ist.

Aus Fertigungsgründen vorteilhaft können der erste und der zweite Schwimmerkörper identisch ausgebildet sein. Dann reicht es aus, nur einen Schwimmerkörper zu fertigen, der allein durch seine Anordnung an der Ventilanordnung ein erster oder ein zweite Schwimmerkörper ist. Bevorzugt sind der erste und der zweite Schwimmerkörper um eine zur Auftriebsachse parallele Überführungsachse verdreht angeordnet, um sicherzustellen, dass deren Auftriebsvolumenabschnitte jeweils in einem anderen Raumbereich des Füllvolumens des die Ventilanordnung tragenden Behälters angeordnet sind. Dann kann somit durch Drehung um die Überführungsachse, gegebenenfalls auch unter zusätzlicher Verschiebung, der eine Schwimmerkörper virtuell in den anderen übergeführt werden. Alternativ kann der Schwimmerköper einen Auftriebsvolumenabschnitt und einen separaten Verbindungsabschnitt umfassen oder aus diesen Komponenten bestehen, wobei der Verbindungsabschnitt an dem Auftriebsvolumenabschnitt befestigbar bzw. mit dem Auftriebsvolumenabschnitt verbindbar ist. Der Schwimmerkörper kann somit aus wenigstens dem Verbindungsabschnitt und dem Auftriebsvolumenabschnitt gebaut sein. Dies hat den Vorteil, dass der Verbindungsabschnitt immer in der gleichen Form hergestellt werden kann und zur Anwendung für einen zweiten Hohlkörper um eine Achse um 180 Grad gedreht werden kann.

In einer ersten möglichen Ausführungsform kann die Ventilsitzformation genau einen Ventilsitz aufweisen und kann die Ventilkörperformation genau einen Ventilkörper aufweisen, wobei der genau eine Ventilkörper durch jeden einzelnen Schwimmerkörper in die Verschlussstellung verbracht werden kann. Eine solche Ventilbaugruppe ist beispielsweise aus der oben bereits genannten EP 2 119 466 A1 bekannt. In einer zweiten möglichen Ausführungsform kann die Ventilsitzformation einen ersten Ventilsitz und mit Abstand von diesem einen zweiten Ventilsitz umfassen, wobei beide Ventilsitze von dem Kanal durchsetzt sind. Folglich umfasst in dieser zweiten Ausführungsform die Ventilkörperformation einen ersten Ventilkörper und einen relativ zu diesem beweglichen zweiten Ventilkörper. In diesem Fall ist der erste Ventilkörper mit dem ersten Schwimmerkörper zur gemeinsamen Bewegung gekoppelt und an den ersten Ventilsitz in körperliche Anlage bringbar. Ebenso ist der zweite Ventilkörper mit dem zweiten Schwimmerkörper zur gemeinsamen Bewegung gekoppelt und an den zweiten Ventilsitz in körperliche Anlage bringbar. Eine derart ausgestaltete Ventilbaugruppe ist aus der oben bereits genannten US 5,445143 bekannt. Die erste Ausführungsform hat den Vorteil identischer Schließkräfte für beide Schwimmerkörper. Die zweite Ausführungsform hat den Vorteil, dass jeder Schwimmerkörper dauerhaft mit dem ihm zugeordneten Ventilkörper verbunden sein kann.

Die vorliegende Erfindung betrifft auch eine Befeuchtungsvorrichtung für eine Beatmungsvorrichtung, umfassend einen Behälter mit einem Füllvolumen, wobei der Behälter eine Einlassöffnung aufweist, durch welche Beatmungsgas in das Füllvolumen einleitbar ist, und eine Auslassöffnung aufweist, durch welche Beatmungsgas aus dem Füllvolumen ausleitbar ist. Die Befeuchtungsvorrichtung weist eine Ventilanordnung auf, die wie oben beschrieben ausgebildet ist. Der Kanal der Ventilanordnung ist dabei ein Zuleitungskanal zur Einleitung von Flüssigkeit in den Behälter.

Das Füllvolumen wird folglich von Beatmungsgas durchströmt und nimmt dabei verdunstete oder verdampfte Flüssigkeit unter Erhöhung seiner Feuchtigkeit in Richtung zum Patienten mit. Zur besseren Einstellbarkeit der Befeuchtung des Beatmungsgases im Füllvolumen ist wenigstens ein Wandabschnitt, vorzugsweise ein Boden, des Behälters aus einem Material mit höherer Wärmeleitfähigkeit als der übrige Behälter ausgebildet. Bevorzugt ist ein Großteil der Behälterwand aus Kunststoff geformt. Der Wandabschnitt mit höherer Wärmeleitfähigkeit ist bevorzugt aus Metall gebildet. Der Wandabschnitt mit höherer Wärmeleitfähigkeit kann so mit einer, vorzugsweise in ihrer Leistung steuerbaren, Wärmequelle in wärmeübertragenden Kontakt gebracht werden, sodass durch die Wärmequelle mittels des Wandabschnitts mit höherer Wärmeleitfähigkeit Wärme in die Flüssigkeit im Füllvolumen eingetragen und so die zeitliche Verdunstungsrate dieser Flüssigkeit betragsmäßig verändert werden kann.

Der Behälter weist einen Behälterboden und eine vom Behälterboden abstehende Seitenwandanordnung auf. Für ein erwünschtes Verschließen des Kanals bei einem unerwünschten Verkippen des Behälters ist es vorteilhaft, wenn wenigstens ein Auftriebsvolumenabschnitt näher an der Seitenwandanordnung angeordnet ist als in einem zentralen Bereich des Füllvolumens des Behälters, da sich beim Verkippen des Behälters in das Füllvolumen eingefüllte Flüssigkeit in der Regel in einem Randbereich nahe der Seitenwandanordnung akkumuliert. Daher gilt bevorzugt für wenigstens einen, vorzugsweise für beide Schwimmerkörper, dass der Abstand des Auftriebsvolumenabschnitts des einen Schwimmerkörpers zu dem ihm nächstgelegenen Abschnitt der Seitenwandanordnung kürzer ist als der Abstand zum Auftriebsvolumenabschnitt des jeweils anderen Schwimmerkörpers.

Bevorzugt steht ein substantieller Teil des Füllvolumens des Behälters für die Durchströmung mit Beatmungsgas und für die Durchmischung des strömenden Beatmungsgases mit verdunsteter bzw. verdampfter Flüssigkeit zur Verfügung. Daher beträgt bevorzugt das von den beiden Schwimmerkörpern eingenommene Volumen nicht mehr als 20 %, vorzugsweise nicht mehr als 15 % des Füllvolumens des Behälters.

Wie oben bereits ausgeführt ist, sind bevorzugt die beiden Schwimmerkörper funktionell gleichwertig, was sich bei der Befeuchtungsvorrichtung dadurch zeigt, dass bei bestimmungsgemäßem Gebrauch mit parallel zur Schwerkraftwirkungsrichtung orientierter Auftriebsachse der erste und der zweite Schwimmerkörper derart ausgebildet und angeordnet sind, dass sich bei Verwendung von demineralisiertem Wasser mit einer Temperatur von 20 °C als Bezugsflüssigkeit zur Befüllung des Behälters die Füllmenge, die notwendig ist, damit der erste Schwimmkörper seine Auftriebsstellung erreicht, von der Füllmenge, die notwendig ist, damit der zweite Schwimmerkörper seine Auftriebsstellung erreicht, bezogen auf die größere der beiden Füllmengen um nicht mehr als 10 %, vorzugsweise um nicht mehr als 5 %, unterscheidet.

Die vorliegende Erfindung wird nachfolgend anhand der beiliegenden Zeichnungen näher erläutert. Es stellt dar:
- Fig. 1:: eine grobschematische perspektivische Ansicht einer Beatmungsvorrichtung mit einer erfindungsgemäßen Ausführungsform einer Befeuchtungsvorrichtung,
- Fig. 2:: eine grobschematische perspektivische Ansicht der Befeuchtungsvorrichtung von Figur 1,
- Fig. 3:: die Befeuchtungsvorrichtung von Figur 2 mit teilweise geschnittener Seitenwandanordnung und teilweise geschnittener Ventilbaugruppe,
- Fig. 4:: eine erfindungsgemäße Ausführungsform einer Ventilanordnung der vorliegenden Anmeldung, wie sie beispielhaft in der Befeuchtungsvorrichtung der Figuren 2 und 3 verwendet ist, mit den Schwimmerkörpern jeweils in der Auftriebsstellung,
- Fig. 5:: die Ventilanordnung von Figur 4 in der Absenkstellung als Bezugszustand und
- Fig. 6:: die Befeuchtungsvorrichtung der Figuren 2 und 3 bei Betrachtung von unten unter Weglassung des Bodens der Vorrichtung.

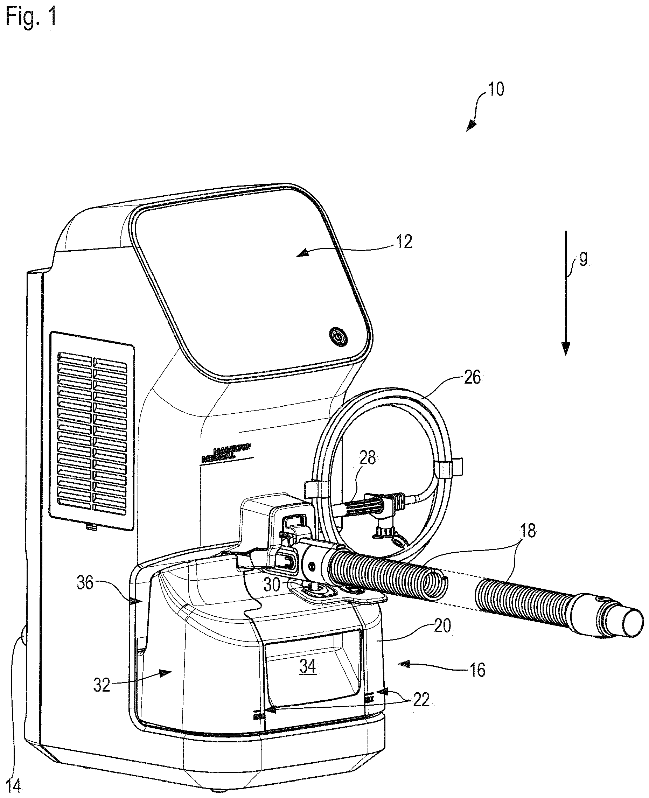

In Figur 1 ist eine Beatmungsvorrichtung allgemein mit 10 bezeichnet. Die Beatmungsvorrichtung 10 weist einen Touchscreen 12 als Eingabe/Ausgabe-Vorrichtung auf, welche mit einer im Gehäuse der Beatmungsvorrichtung 10 angeordneten Steuervorrichtung in Datenübertragungsverbindung steht. Die Beatmungsvorrichtung 10 weist in ihrem Gehäuse ein Gebläse auf, mit welchem Umgebungsluft als Beatmungsgas über einen rückseitigen Einlass 14 angesaugt wird. Alternativ kann das Beatmungsgas jedoch auch aus diversen Gasen, die mittels Anschlüssen mit der Beatmungsvorrichtung verbunden sind, gemischt werden.

In der unteren vorderen Hälfte der Beatmungsvorrichtung 10 ist eine Befeuchtungsvorrichtung 16 angeordnet, die der Befeuchtung des Beatmungsgases dient, bevor dieses über einen Beatmungsschlauch 18 zu einem Patienten hin gefördert wird.

Die Befeuchtungsvorrichtung 16 umfasst einen Behälter 20, in welchen Flüssigkeit, in der Regel Wasser, eingefüllt werden kann. Die in den Behälter 20 eingefüllte Flüssigkeit verdunstet bzw. verdampft dort, wobei sich das den Behälter 20 durchströmende Beatmungsgas mit der verdunsteten bzw. verdampfen Flüssigkeit vermischt. Dadurch verlässt das Beatmungsgas den Behälter 20 durch den Beatmungsschlauch 18 mit betragsmäßig größerer absoluter Feuchte als es in den Behälter 20 hinein gefördert wurde. Der Beatmungsschlauch 18 ist in Figur 1 verkürzt nur mit seinen beiden Längsendabschnitten dargestellt. Ein Mittelabschnitt des Beatmungsschlauchs 18 ist nicht dargestellt.

In Figur 1 befindet sich die Beatmungsvorrichtung 10 und mit ihr die Befeuchtungsvorrichtung 16 in einem bestimmungsgemäßen Betriebszustand, in welchem eine ebene Aufstandsfläche der Beatmungsvorrichtung 10 orthogonal zur Schwerkraftwirkungsrichtung g orientiert ist.

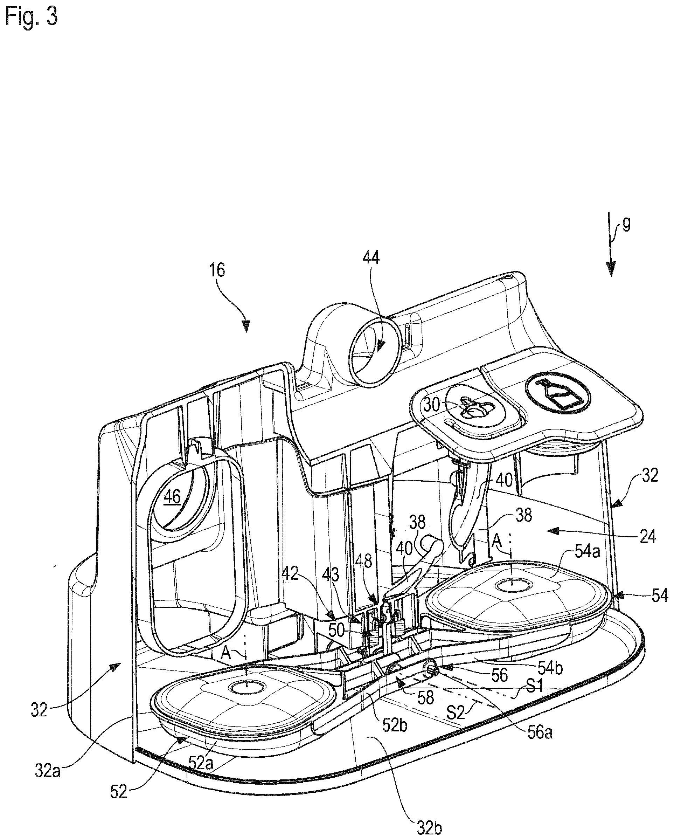

Eine Markierung 22 an der Vorderseite des Behälters 20 zeigt den maximalen Füllstand an, welchen die Flüssigkeit im Füllvolumen 24 (siehe Figur 3) des Behälters 20 nicht überschreiten soll.

In das Füllvolumen 24 des Behälters 20 ist Flüssigkeit aus einem in Figur 1 nicht dargestellten Vorrat über eine Lieferleitung 26 einleitbar. Die Lieferleitung 26 ist in Figur 1 außer Betrieb beispielhaft als Schlauchrolle dargestellt. Die Lieferleitung 26 weist an ihrem dem Behälter 20 fernen Längsende eine Kopplungsformation 28, beispielsweise eine Einstech-Kopplung 28, auf, mit welcher das Lumen der Lieferleitung 26 mit einer bevorrateten Flüssigkeit verbunden werden kann. Die Kopplungsformation 28 gestattet in an sich bekannter Weise die Beigabe von Zusatzstoffen, wie etwa Medikamenten, zu der in der Lieferleitung 26 strömenden Flüssigkeit.

An ihrem dem Behälter 20 nahen Längsende ist die Lieferleitung 26 mit einer Durchgangsöffnung 30 in der Behälterwand 32 gekoppelt, sodass aus dem Flüssigkeitsvorrat in der Regel schwerkraftgetrieben durch die Lieferleitung 26 strömende Flüssigkeit in das Füllvolumen 24 des Behälters 20 gelangen kann.

Über eine Griffmulde 34 an der Vorderseite des Behälters 20 ist der Behälter 20 aus einer Einsetz-Ausnehmung 36 an der Beatmungsvorrichtung 10 entnehmbar und wieder in diese einsetzbar.

Wie Figur 3 zeigt, schließt auf der Innenseite der Behälterwand 32 an der Durchgangsöffnung 30 ein Kanalbauteil 38 an, in welchem ein Kanal 40 ausgebildet ist, welcher die von der Lieferleitung 26 gelieferte Flüssigkeit zu einer Ventilanordnung 42 und, abhängig vom Betriebszustand der Ventilanordnung 42, durch diese hindurch fördert. Das Kanalbauteil 38 ist in Figur 3 längs einer zur Auftriebsachse A parallelen und zu den weiter unten erläuterten Schwenkachsen S1 und S2 orthogonalen Schnittebene geschnitten dargestellt, um den Kanal 40 zu zeigen. In einem Mittelbereich des Kanalbauteils 38 ist aufgrund von dessen Krümmung um eine zur Auftriebsachse A parallele Krümmungsachse aus diesem ein Stück herausgeschnitten.

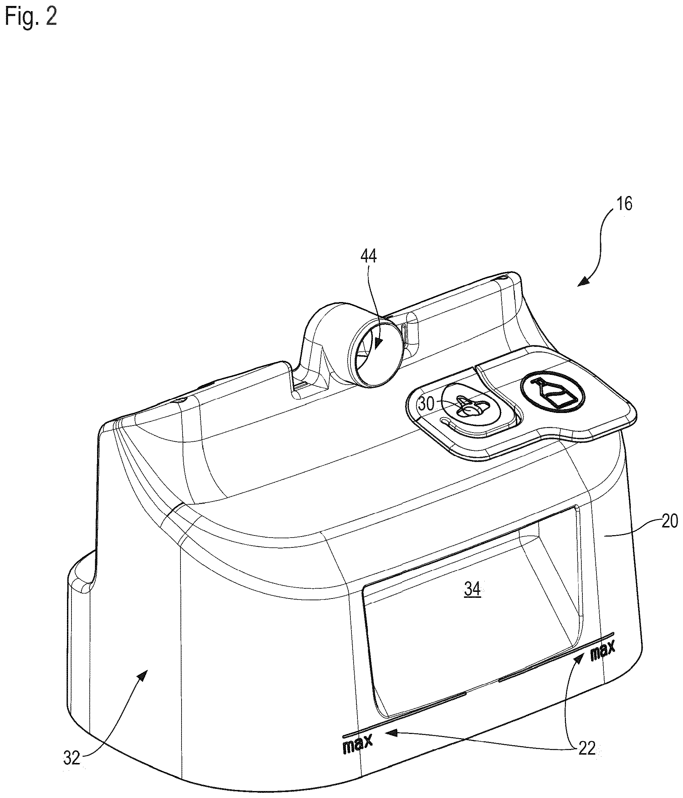

In den Figuren 2 und 3 ist außerdem die Auslassöffnung 44 dargestellt, durch welche hindurch das das Füllvolumen 24 durchströmende Beatmungsgas aus dem Behälter 20 austritt. Figur 3 zeigt die Einlassöffnung 46, durch welche hindurch das Beatmungsgas in das Füllvolumen 24 einströmt.

Die Behälterwand 32 umfasst eine Seitenwandanordnung 32a und einen im Wesentlichen ebenen Boden 32b. Die Seitenwandanordnung 32a ist bevorzugt urformend im Spritzgussverfahren aus thermoplastischem Kunststoff hergestellt. Der Boden 32b ist aus Metall und weist eine höhere Wärmeleitfähigkeit auf als in die Seitenwandanordnung 32a. Die Einsetz-Ausnehmung 36 der Beatmungsvorrichtung 10 weist an ihrer Unterseite eine Heizvorrichtung auf, welche dann, wenn die Befeuchtungsvorrichtung 16 in die Einsetz-Ausnehmung 36 eingesetzt ist, in Wärmeübertragungskontakt mit dem, vorzugsweise metallischen, Boden 32b steht, um Wärme mit möglichst geringer Zeitverzögerung und unter möglichst geringen Verlusten in die sich im betriebsbereiten Bezugszustand am Boden 32b akkumulierende Flüssigkeit einzutragen. Im betriebsbereiten Bezugszustand der Befeuchtungsvorrichtung 16 ist der Boden 32b des Behälters 20 im Wesentlichen orthogonal zur Schwerkraftwirkungsrichtung g orientiert, sodass sich ein Flüssigkeitsspiegel einer in das Füllvolumen 24 eingefüllten Flüssigkeit im Wesentlichen parallel zum Boden 32b ausrichtet.

Die Ventilanordnung 42 umfasst eine Ventilbaugruppe 43 mit einer vom Kanal 40 durchsetzten Ventilsitzformation 48 und mit einer relativ zur Ventilsitzformation 48 beweglichen Ventilkörperformation 50, die mit der Ventilsitzformation 48 zusammenwirkt, um den die Ventilsitzformation 48 durchsetzenden Kanal wahlweise zu verschließen oder zu öffnen.

Im bestimmungsgemäßen Betriebszustand durchsetzt der Kanal 40 die Ventilsitzformation 48 parallel zur Schwerkraftwirkungsrichtung g, weshalb die Ventilkörperformation 50 bevorzugt ebenfalls parallel zur Schwerkraftwirkungsrichtung g relativ zur Ventilsitzformation 48 beweglich ist. Grundsätzlich ist bevorzugt die Ventilkörperformation 50 parallel zu der Richtung relativ zur Ventilsitzformation 48 beweglich, in welcher der Kanal 40 die Ventilsitzformation 48 durchsetzt.

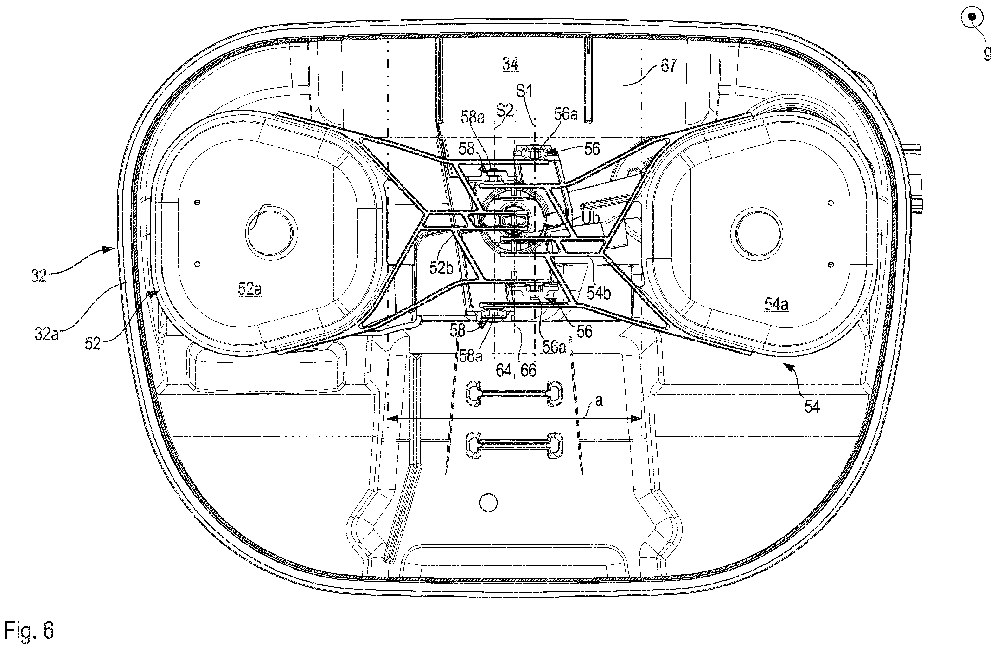

Die Ventilanordnung 42 weist als Aktuatoren der Ventilkörperformation 50 einen ersten Schwimmerkörper 52 und einen zweiten Schwimmerkörper 54 auf. Die beiden Schwimmerkörper 52 und 54 sind identisch ausgebildet und lediglich unterschiedlich orientiert im Füllvolumen 24 angeordnet. Die beiden Schwimmerkörper sind um eine zum Boden 32b orthogonale Überführungsachse Ub (s. Fig. 6) durch Drehung um 180° virtuell ineinander überführbar.

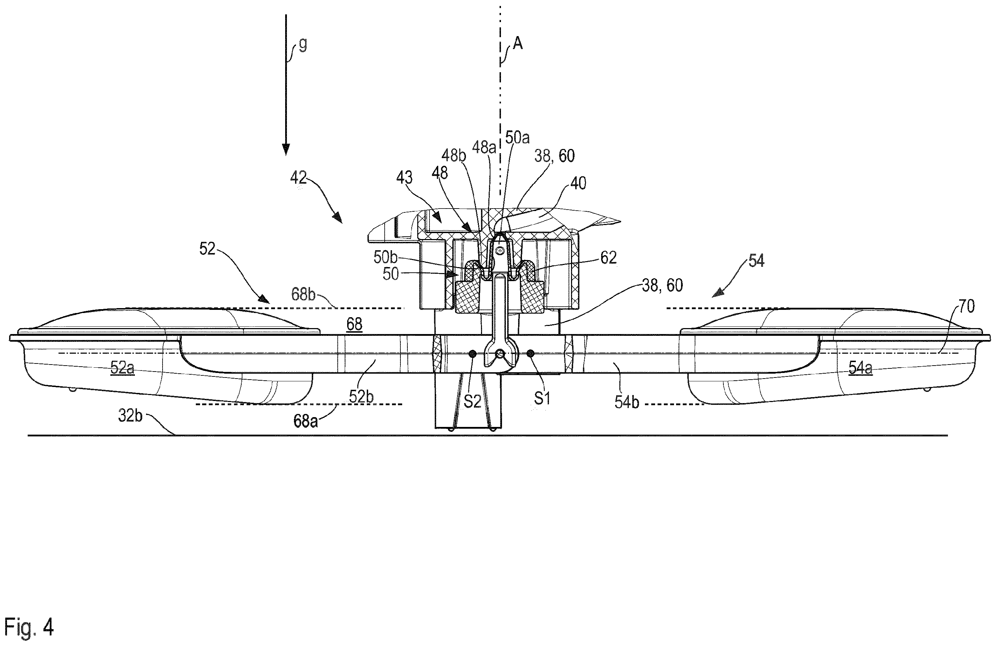

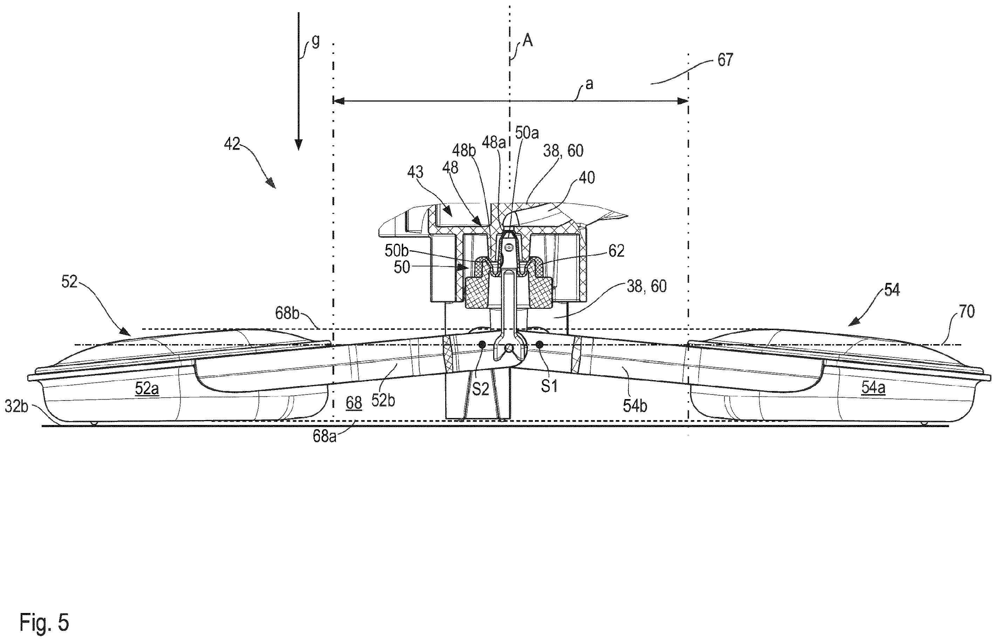

Der erste Schwimmerkörper 52 ist um eine erste Schwenkachse S1 an einem ersten Gelenk 56 gelenkig gelagert. Der erste Schwimmerkörper 52 weist mit Abstand von der ersten Schwenkachse S1 einen Auftriebsvolumenabschnitt 52a auf, welcher über einen Verbindungsabschnitt 52b mit dem Gelenk 56 verbunden ist. Der Verbindungsabschnitt 52b ist als fachwerkartiger Stegabschnitt ausgebildet. Der Auftriebsvolumenabschnitt 52a nimmt das Hauptvolumen des ersten Schwimmerkörpers 52 ein und sorgt in Wechselwirkung mit einer im Füllvolumen 24 aufgenommenen Flüssigkeit für den größten Teil des durch den ersten Schwimmerkörper 52 bereitgestellten Auftriebs. Der Auftriebsvolumenabschnitt 52a bewegt sich zwischen seinen in den Figuren 4 und 5 dargestellten Betriebsstellungen: Auftriebsstellung (Figur 4) und Absenkstellung (Figur 5), längs einer Auftriebsachse A, die im bestimmungsgemäß betriebsbereiten Zustand der Ventilanordnung 42 parallel zur Schwerkraftwirkungsrichtung g verläuft. Da der Auftriebsvolumenabschnitt 52a tatsächlich zwangsgebunden eine kreisförmige Bahn um die erste Schwenkachse S1 ausführt, weist die Trajektorie des Auftriebsvolumenabschnitts 52a zwischen seinen Betriebsstellungen auch eine Bewegungskomponente orthogonal zur Auftriebsachse A auf, die jedoch erstens keinen Beitrag zur Verlagerung der Ventilkörperformation leistet und die zweitens betragsmäßig im Vergleich zur Bewegungskomponente längs der Auftriebsachse A vernachlässigbar gering ist.

Wie in Figur 3 gut zu erkennen ist, ist der erste Auftriebsvolumenabschnitt 52a nahe bei dem ihm nächstgelegenen Abschnitt der Seitenwandanordnung 32a gelegen. Der erste Auftriebsvolumenabschnitt 52a ist näher bei dem ihm nächstgelegenen Abschnitt der Seitenwandanordnung 32a gelegen als bei einem den Boden 32b in seinem Flächenschwerpunkt durchsetzenden Mittellot, welches orthogonal zum Boden 32b und damit parallel zur Auftriebsachse A verläuft. Das Mittellot verläuft in Figur 3 in einer zur Auftriebsachse A parallelen Ebene, welche in der Abstandsmitte zwischen der ersten Schwenkachse S1 und der zweiten Schwenkachse S2 gelegen ist. Erst recht befindet sich jeder Auftriebsvolumenabschnitt 52a und 54a jeweils näher an dem ihm nächstgelegenen Abschnitt der Seitenwandanordnung 32a als an dem jeweils anderen Auftriebsvolumenabschnitt 52a bzw. 54a.

Um die zweite Schwenkachse S2 ist der zweite Schwimmerkörper 54 in analoger Weise schwenkbar wie der erste Schwimmerkörper 54 um die erste Schwenkachse S1. Wegen der identischen Ausbildung weist der zweite Schwimmerkörper 54 einen Auftriebsvolumenabschnitt 54a auf, welcher durch einen als fachwerkartige Stegformation ausgebildeten Verbindungsabschnitt 54b mit einem zweiten Gelenk 58 verbunden ist. "Fachwerkartig" bedeutet in diesem Zusammenhang, dass die Stegformation miteinander verbundene Längs- und Querstreben aufweist. Die längs- und Querstreben bilden dabei zur Erzielung einer statisch stabilen Stegformation entweder Dreiecks- oder Vierecks-Fachwerke.

Anhand von Figur 4 wird der Aufbau der Ventilbaugruppe 43 näher erläutert. Die Ventilsitzformation 48 weist einen ersten Ventilsitz 48a und einen längs der Auftriebsachse A mit Abstand von diesem ausgebildeten zweiten Ventilsitz 48b auf. Die beiden Ventilsitze 48a und 48b sind an dem Kanalbauteil 38 ausgebildet, welches auch ein Ventilgehäuse 60 bildet. Der erste Ventilsitz 48a weist beispielhaft eine negativ-konische Anlagefläche auf, der zweite Ventilsitz 48b eine positiv-konische Anlagefläche. Beide Ventilsitze 48a und 48b sind von dem Kanal 40 durchsetzt.

Die Ventilkörperformation 50 weist einen ersten Ventilkörper 50a auf, welche im dargestellten Beispiel stiftförmig ausgebildet ist, und weist einen relativ zu diesem beweglichen zweiten Ventilkörper 50b auf, welcher im dargestellten Beispiel rohrförmig ausgebildet ist. Der erste Ventilkörper 50a weist eine positiv-konische Anlagefläche zur Wechselwirkung mit der negativ-konischen Anlagefläche des ersten Ventilsitzes 48a auf. Der zweite Ventilkörper 50b weist eine negativ-konische Anlagefläche zur Wechselwirkung mit der positiv-konischen Anlagefläche des zweiten Ventilsitzes 48b auf. Die Ventilkörperformation 50 weist außerdem eine weichelastische Membran 62 auf, welche zur Erhöhung der Dichtigkeit der Ventilbaugruppe 43 in der in Figur 4 gezeigten Verschlussstellung beide Ventilkörper 50a und 50b überspannt. Beide Ventilkörper 50a und 50b sind ganz überwiegend längs der Auftriebsachse A verlagerbar.

Der erste Ventilkörper 50a ist gelenkig mit dem ersten Schwimmerkörper 52, im dargestellten Beispiel mit dessen Verbindungsabschnitt 52b, gekoppelt, sodass eine Bewegung des ersten Verbindungsabschnitts 52b längs der Auftriebsachse A eine Verlagerung des ersten Ventilkörpers 50a zwischen der in Figur 4 gezeigten Verschlussstellung und der in Figur 5 gezeigten Durchlassstellung bewirkt.

Ebenso ist der zweite Ventilkörper 50b gelenkig mit dem zweiten Schwimmerkörper 54, im dargestellten Beispiel mit dessen Verbindungsabschnitt 54b, gekoppelt, sodass eine Bewegung des zweiten Verbindungsabschnitts 54b längs der Auftriebsachse A eine Verlagerung des zweiten Ventilkörpers 50b zwischen der in Figur 4 gezeigten Verschlussstellung und der in Figur 5 gezeigten Durchlassstellung bewirkt. Dabei reicht es aus, wenn einer der beiden Ventilkörper 50a oder 50b in seine Verschlussstellung verlagert wird, um den Kanal 40 für eine Durchströmung mit Flüssigkeit zu verschließen.

Die Ventilanordnung 42 ist derart gestaltet, dass die Gelenkachsen 64 und 66 der Verbindungen (siehe Figur 6) des ersten Ventilkörpers 50a mit dem ersten Verbindungsabschnitt 52b bzw. des zweiten Ventilkörpers 50b mit dem zweiten Verbindungsabschnitt 54b dann, wenn sich jeder Schwimmerkörper 52 und 54 in wenigstens einer identischen Endstellung aus Absenkstellung und Auftriebsstellung befindet, koaxial verlaufen. Die koaxialen Gelenkachsen 64 und 66 sind dann äquidistant zu den Schwenkachsen S1 und S2 angeordnet.

Weiter ist die Ventilanordnung 42 derart gestaltet, dass die Schwimmerkörper 52 und 54 im Wesentlichen diametral zu der parallelen Auftriebsachse (A) angeordnet sind. In anderen Worten ausgedrückt: wenn die Gelenkachsen 64 und 66 eine zur Auftriebsachse (A) parallele virtuelle Bezugsebene aufspannen, befindet sich ein Schwimmerkörper auf einer Seite der virtuellen Bezugsebene und der andere Schwimmerkörper auf der anderen Seite der virtuellen Bezugsebene.

Von den im Abstand a (s. Fig. 5) voneinander angeordneten Auftriebsvolumenabschnitten 52a und 54a liegt der erste Auftriebsvolumenabschnitt 52a näher an der zweiten Schwenkachse S2 und liegt der zweite Auftriebsvolumenabschnitt 54a näher an der ersten Schwenkachse S1. Die Kopplungsstellen der beiden Ventilkörper 50a und 50b mit den Schwimmerkörpern 52 und 54 liegen im Erstreckungsbereich der beiden Verbindungsabschnitte 52b und 54b, welcher zwischen den beiden Schwenkachsen S1 und S2 gelegen ist.

Die Gelenke 56 und 58 sind zwischen den jeweiligen Schwimmerkörpern 52 bzw. 54 und dem Ventilgehäuse 60 gebildet. Im dargestellten Beispiel ist an den jeweiligen Schwimmerkörpern 52 bzw. 54 als Spritzgussbauteile ein Wellenstumpf als schwimmerkörperseitiger Gelenkabschnitt ausgebildet (siehe Wellenstumpf 56a in Figur 3 sowie die Wellenstümpfe 56a und 58a in Figur 6). Im Ventilgehäuse 60 ist als lagerseitiger Gelenkabschnitt eine den jeweiligen Wellenstumpf aufnehmende Ausnehmung ausgebildet.

In Figur 5 ist die Ventilanordnung 42 in ihrer Absenkstellung und damit in dem zu ihrer Erläuterung in der Beschreibungseinleitung verwendeten Bezugszustand dargestellt. Die Auftriebsvolumenabschnitte 52a und 54a weisen an der Außenseite ihrer Böden kleine Vorsprünge auf, mit welchen die Auftriebsvolumenabschnitte 52a und 54a auf dem vorzugsweise ebenen Boden 32b des Behälters 20 aufliegen. Die kleinen Vorsprünge sorgen dafür, dass die Auftriebsvolumenabschnitte 52a und 54a auch in der Absenkstellung von Flüssigkeit im Füllvolumen 24 unterspült werden können, sodass bereits kleinste Flüssigkeitsmengen einen Auftrieb an den Auftriebsvolumenabschnitten 52a und 54a bewirken.

Zwischen den Auftriebsvolumenabschnitten 52a und 54a befindet sich ein Körper-Abstandsbereich 67, in welchem im dargestellten Ausführungsbeispiel beide Gelenke 56 und 58 angeordnet sind. Der Körper-Abstandsbereich 67 erstreckt sich orthogonal zur Auftriebsachse A über den Abstand a hinweg, der zwischen den Auftriebsvolumenabschnitten 52a und 54a besteht. Durch die Anordnung der Auftriebsvolumenabschnitte 52a und 54a im Abstand a voneinander kann jeder Auftriebsvolumenabschnitt 52a und 54a an einem Randbereich des Füllvolumens 24, also nahe einem Abschnitt der Seitenwandanordnung 32a, angeordnet werden, was die Sensitivität der Ventilanordnung 42 bezüglich eines Verkippens der Befeuchtungsvorrichtung 16 um eine sowohl zur Auftriebsachse A als auch zur Richtung des Abstands a orthogonale Kippachse in vorteilhafter Weise erheblich erhöht.

Dann, wenn sich beide Schwimmerkörper in der Absenkstellung befinden, befinden sich die Gelenke 56 und 58 sowie die durch sie definierten Schwenkachsen S1 und S2 in einem gemeinsamen Höhenerstreckungsbereich 68 der Auftriebsvolumenabschnitte 52a und 54a, welcher nach unten durch eine Ebene 68a und nach oben durch eine Ebene 68b begrenzt ist. Die beiden Ebenen 68a und 68b sind zur Auftriebsachse A orthogonale Schmiegeebenen an die Unterseiten bzw. an die Oberseiten der Auftriebsvolumenabschnitte 52a und 54a. Dieses Anordnungsverhältnis von Gelenken 56 und 58 zu den durch sie zwangsgeführten Auftriebsvolumenabschnitten 52a und 54a sorgt für eine vorteilhafte Kinematik der Auftriebsvolumenabschnitte 52a und 54a mit einer wesentlich größeren Bewegungskomponente längs der Auftriebsachse A als orthogonal zu dieser. Aus demselben Grunde einer Erzielung einer vorteilhaften Kinematik der Auftriebsvolumenabschnitte 52a und 54a sind die Schwenkachsen S1 und S2 in einer gemeinsamen virtuellen Ebene 70 angeordnet, welche die Auftriebsvolumenabschnitte 52a und 54a wenigstens dann schneidet, wenn diese sich in der Absenkstellung befinden. Wie Figur 4 jedoch zeigt, ist dies auch dann der Fall, wenn sich die Auftriebsvolumenabschnitte 52a und 54a in der Auftriebsstellung befinden. Die virtuelle Erstreckungsebene 70 ist orthogonal zur Auftriebsachse A und damit orthogonal zur Zeichenebene der Figuren 4 und 5 orientiert.

Da die individuellen Höhenerstreckungsbereiche der beiden Auftriebsvolumenabschnitte 52a und 54a hinsichtlich Abmessung und Lage identisch sind, reichen zur Bestimmung des mit den individuellen Höhenerstreckungsbereichen identischen gemeinsamen Höhenerstreckungsbereichs 68 genau zwei Ebenen aus, eine obere und eine untere. Falls die individuellen Höhenerstreckungsbereiche der beiden Auftriebsvolumenabschnitte 52a und 54a hinsichtlich Abmessung oder/und Lage unterschiedlich sind, sind in analoger Weise die individuellen Höhenerstreckungsbereiche eines jeden Auftriebsvolumenabschnitts 52a und 54a zu bestimmen. Der gemeinsame Höhenerstreckungsbereich 68 ist die Schnittmenge der individuellen Höhenerstreckungsbereiche.

Dann, wenn die Befeuchtungsvorrichtung 16 und damit die Ventilanordnung 42 in einer beliebigen Kipprichtung um eine zu den Schwenkachsen S1 und S2 parallelen Kippachse gekippt wird, verlagert sich Flüssigkeit zu dem durch die Kippbewegungen abgesenkten Seitenwandabschnitt hin, wodurch sich Flüssigkeit in einem Anordnungsbereich einer der beiden Auftriebsvolumenabschnitte 52a und 54a akkumuliert und so den betreffenden Auftriebsvolumenabschnitt und mit ihm den gesamten Schwimmerkörper in die Auftriebsstellung verlagern kann. Hierdurch kann verhindert werden, dass Flüssigkeit dann in das Füllvolumen der Befeuchtungsvorrichtung 16 nachfließt, wenn diese sich in einer unerwünscht verkippten Position befindet. Dies gilt auch für eine Kippbewegung um eine Kippachse, welche nicht vollständig mit einer der Schwenkachsen parallel ist, solange ihr Verlaufsanteil längs einer der Schwenkachsen größer ist als orthogonal zu dieser.

Abweichend von der Darstellung im Ausführungsbeispiel müssen die Schwenkachsen S1 und S2 nicht zueinander parallel sein. Vorzugsweise liegen sie dennoch in einer gemeinsamen Ebene.

## Patentansprüche

1. Schwimmergesteuerte Ventilanordnung (42), umfassend eine Ventilbaugruppe (43) mit einem Kanal (40), einer von dem Kanal durchsetzten Ventilsitzformation (48) und einer Ventilkörperformation (50), wobei die Ventilkörperformation (50) relativ zur Ventilsitzformation (48) verlagerbar ist zwischen einer Verschlussstellung, in welcher der Kanal (40) durch körperliche Anlage der Ventilkörperformation (50) an der Ventilsitzformation (48) verschlossen ist, und einer Durchlassstellung, in welcher die Ventilkörperformation (50) mit Abstand von der Ventilsitzformation (48) angeordnet ist, so dass der Kanal (40) durchströmbar ist, wobei die Ventilanordnung (42) weiter umfasst: einen ersten Schwimmerkörper (52) mit einem ersten Auftriebsvolumenabschnitt (52a) und einen zweiten Schwimmerkörper (54) mit einem zweiten Auftriebsvolumenabschnitt (54a), wobei der erste Schwimmerkörper (52) an einem ersten Gelenk (56) schwenkbar angelenkt ist und wobei der zweite Schwimmerkörper (54) an einem zweiten Gelenk (58) schwenkbar angelenkt ist, so dass jeder einzelne Schwimmerkörper (52, 54) im bestimmungsgemäßen Betrieb längs einer zur Schwerkraftwirkungsrichtung (g) parallelen Auftriebsachse (A) bewegbar ist zwischen einer Absenkstellung und einer Auftriebsstellung, wobei der erste (52) und der zweite Schwimmerkörper (54) jeweils derart mit der Ventilkörperformation (50) gekoppelt ist, dass sich die Ventilkörperformation (50) dann in der Verschlussstellung befindet, wenn sich wenigstens einer der Schwimmerkörper (52, 54) in der Auftriebsstellung befindet, wobei jeder einzelne Schwimmerkörper (52, 54) dann, wenn in seinem Anordnungsbereich ausreichend Flüssigkeit akkumuliert ist, in die Auftriebsstellung auftreibt und den Kanal (40) verschließt, und sich in der Durchlassstellung befindet, wenn sich beide Schwimmerkörper (52, 54) in der Absenkstellung befinden,
**dadurch gekennzeichnet, dass**, bei Betrachtung der beiden Schwimmerkörper (52, 54) in ihrer jeweiligen Absenkstellung als einem Bezugszustand, die jeweiligen Auftriebsvolumenabschnitte (52a, 54a) der beiden Schwimmerkörper (52, 54) mit einem zur Auftriebsachse (A) orthogonalen Abstand (a) voneinander angeordnet sind, wobei in einem Körper-Abstandsbereich (67) zwischen beiden Auftriebsvolumenabschnitten (52a, 54a) wenigstens ein Gelenk (56, 58) gelegen ist oder/und wobei in einem Gelenk-Abstandsbereich zwischen beiden Gelenken (56, 58) wenigstens ein Auftriebsvolumenabschnitt (52a, 54a) gelegen ist.

2. Ventilanordnung (42) nach Anspruch 1,
**dadurch gekennzeichnet, dass** im Bezugszustand in dem Körper-Abstandsbereich (67) beide Gelenke (56, 58) gelegen sind.

3. Ventilanordnung (42) nach Anspruch 1 oder 2,
**dadurch gekennzeichnet, dass** wenigstens ein in dem Körper-Abstandsbereich (67) gelegenes Gelenk (56, 58) in einem sich längs der Auftriebsachse (A) erstreckenden Höhenerstreckungsbereich (68) angeordnet ist, in welchem sich im Bezugszustand auch die beiden Auftriebsvolumenabschnitte (52a, 54a) erstrecken.

4. Ventilanordnung (42) nach einem der vorhergehenden Ansprüche,
**dadurch gekennzeichnet, dass** eine erste virtuelle Schwenkachse (S1), um welche der erste Schwimmerkörper (52) schwenkbar am ersten Gelenk (56) angelenkt ist, und eine zweite virtuelle Schwenkachse (S2), um welche der zweite Schwimmerkörper (54) schwenkbar am zweiten Gelenk (58) angelenkt ist, in einer gemeinsamen virtuellen Erstreckungsebene (70) gelegen sind, wobei die virtuelle Erstreckungsebene (70) bevorzugt einen Auftriebsvolumenabschnitt (52a, 54a) des ersten (52) und des zweiten Schwimmerkörpers (54) im Bezugszustand schneidet.

5. Ventilanordnung (42) nach Anspruch 4,
**dadurch gekennzeichnet, dass** die virtuelle Erstreckungsebene (70) orthogonal zur Auftriebsachse (A) ausgerichtet ist.

6. Ventilanordnung (42) nach einem der vorhergehenden Ansprüche,
**dadurch gekennzeichnet, dass** die Ventilbaugruppe (43) ein Ventilgehäuse (60) umfasst, an welchem der Kanal (40) ausgebildet ist, wobei jedes Gelenk (56, 58) einen schwimmerkörperseitigen Gelenkabschnitt (56a, 58a) und einen mit dem schwimmerkörperseitigen Gelenkabschnitt (56a, 58a) wechselwirkenden lagerseitigen Gelenkabschnitt umfasst, wobei der lagerseitige Gelenkabschnitt wenigstens eines Gelenks (56, 58), vorzugsweise beider Gelenke (56, 58), am Ventilgehäuse (60) ausgebildet ist.

7. Ventilanordnung (42) nach Anspruch 6,
**dadurch gekennzeichnet, dass** das Ventilgehäuse (60) einstückig oder zweistückig ausgebildet ist.

8. Ventilanordnung (42) nach einem der vorhergehenden Ansprüche, unter Einbeziehung des Anspruchs 4,
**dadurch gekennzeichnet, dass** der erste Auftriebsvolumenabschnitt (52a) näher an der zweiten Schwenkachse (S2) als an der ersten Schwenkachse (S1) gelegen ist oder/und dass der zweite Auftriebsvolumenabschnitt (54a) näher an der ersten Schwenkachse (S1) als an der zweiten Schwenkachse S2) gelegen ist.

9. Ventilanordnung (42) nach Anspruch 8,
**dadurch gekennzeichnet, dass** eine Kopplung des ersten Schwimmerkörpers (52) mit der Ventilkörperformation (50) in einem Bereich zwischen der ersten (S1) und der zweiten Schwenkachse (S2) gelegen ist oder/und dass eine Kopplung des zweiten Schwimmerkörpers (54) mit der Ventilkörperformation (50) in einem Bereich zwischen der ersten (S1) und der zweiten Schwenkachse (S2) gelegen ist.

10. Ventilanordnung (42) nach einem der vorhergehenden Ansprüche,
**dadurch gekennzeichnet, dass** der erste (52) und der zweite Schwimmerkörper (54) identisch ausgebildet sind, wobei bevorzugt der erste (52) und der zweite Schwimmerkörper (54) um eine zur Auftriebsachse (A) parallele Überführungsachse (Ub) relativ zu einander verdreht angeordnet sind.

11. Ventilanordnung (42) nach einem der vorhergehenden Ansprüche,
**dadurch gekennzeichnet, dass** die Ventilsitzformation (48) einen ersten Ventilsitz (48a) und mit Abstand von diesem einen zweiten Ventilsitz (48b) umfasst, wobei beide Ventilsitze (48a, 48b) von dem Kanal (40) durchsetzt sind, und dass die Ventilkörperformation (50) einen ersten Ventilkörper (50a) und einen relativ zu diesem beweglichen zweiten Ventilkörper (50b) umfasst, wobei der erste Ventilkörper (50a) mit dem ersten Schwimmerkörper (52) zur gemeinsamen Bewegung gekoppelt ist und an den ersten Ventilsitz (48a) in körperliche Anlage bringbar ist, und wobei der zweite Ventilkörper (50b) mit dem zweiten Schwimmerkörper (54) zur gemeinsamen Bewegung gekoppelt ist und an den zweiten Ventilsitz (48b) in körperliche Anlage bringbar ist.

12. Befeuchtungsvorrichtung (16) für eine Beatmungsvorrichtung (10), umfassend einen Behälter (20) mit einem Füllvolumen (24), wobei der Behälter (20) eine Einlassöffnung (46) aufweist, durch welche Beatmungsgas in das Füllvolumen (24) einleitbar ist, und eine Auslassöffnung (44) aufweist, durch welche Beatmungsgas aus dem Füllvolumen (24) ausleitbar ist, wobei die Befeuchtungsvorrichtung (16) eine Ventilanordnung (42) nach einem der vorhergehenden Ansprüche aufweist, wobei der Kanal (40) der Ventilanordnung (42) ein Zuleitungskanal zur Einleitung von Flüssigkeit in den Behälter (20) ist.

13. Befeuchtungsvorrichtung (16) nach Anspruch 12,
**dadurch gekennzeichnet, dass** der Behälter (20) einen Behälterboden (32b) und eine vom Behälterboden (32b) abstehende Seitenwandanordnung (32a) aufweist, wobei für wenigstens einen, vorzugsweise für beide, der Schwimmerkörper (52, 54) gilt, dass der Abstand seines Auftriebsvolumenabschnitts (52a, 54a) zu dem ihm nächstgelegenen Abschnitt der Seitenwandanordnung (32b) kürzer ist als der Abstand (a) zum Auftriebsvolumenabschnitt (52a, 54a) des jeweils anderen Schwimmerkörpers (52, 54).

14. Befeuchtungsvorrichtung (16) nach Anspruch 12 oder 13,
**dadurch gekennzeichnet, dass** das von den beiden Schwimmerkörpern (52, 54) eingenommene Volumen nicht mehr als 20 %, vorzugsweise nicht mehr als 15 % des Füllvolumens (24) des Behälters (20) beträgt.

15. Befeuchtungsvorrichtung (16) nach einem der Ansprüche 12 bis 14,
**dadurch gekennzeichnet, dass** bei bestimmungsgemäßem Gebrauch mit parallel zur Schwerkraftwirkungsrichtung (g) orientierter Auftriebsachse (A) der erste (52) und der zweite Schwimmerkörper (54) derart ausgebildet und angeordnet sind, dass sich bei Verwendung von demineralisiertem Wasser mit einer Temperatur von 20 °C als Bezugsflüssigkeit zur Befüllung des Behälters (20) die Füllmenge, die notwendig ist, damit der erste Schwimmkörper (52) seine Auftriebsstellung erreicht, von der Füllmenge, die notwendig ist, damit der zweite Schwimmkörper (54) seine Auftriebsstellung erreicht, bezogen auf die größere der beiden Füllmengen um nicht mehr als 10%, vorzugsweise um nicht mehr als 5 % unterscheidet.

## Claims

1. Float-controlled valve array (42), comprising a valve module (43) with a duct (40), a valve seat formation (48) penetrated through by the duct, and a valve body formation (50), where the valve body formation (50) is displaceable relative to the valve seat formation (48) between a closure position in which the duct (40) is closed through physical abutment of the valve body formation (50) against the valve seat formation (48), and a passage position in which the valve body formation (50) is arranged with a separation from the valve seat formation (48) such that flow through the duct (40) is possible, where the valve array (42) further comprises: a first float body (52) with a first buoyancy volume section (52a) and a second float body (54) with a second buoyancy volume section (54a), where the first float body (52) is articulated in a swiveling manner at a first joint (56) and where the second float body (54) is articulated in a swiveling manner at a second joint (58) such that in normal operation each individual float body (52, 54) is moveable along a buoyancy axis (A) parallel to the gravitational direction (g) between a sinking position and a buoyancy position, where the first (52) and the second float body (54) is each coupled in such a way with the valve body formation (50) that the valve body formation (50) is in the closure position when at least one of the float bodies (52, 54) is in the buoyancy position, wherein each individual float body (52, 54), when sufficient fluid is accumulated in its arrangement region, rises into the buoyancy position and closes the duct (40), and is in the passage position when both float bodies (52, 54) are in the sinking position, **Characterized in that** when regarding the two float bodies (52, 54) in their respective sinking position as a reference state, the respective buoyancy volume sections (52a, 54a) of the two float bodies (52, 54) are arranged with separation (a) from one another which is orthogonal to the buoyancy axis (A), where in a body separation region (67) between the two buoyancy volume sections (52a, 54a) there is located at least one joint (56, 58) and/or where in a joint separation region between two joints (56, 58) there is located at least one buoyancy volume section (52a, 54a).

2. Valve array (42) according to Claim 1,
**Characterized in that** in the reference state both joints (56, 58) are located in the body separation region (67).

3. Valve array (42) according to Claim 1 or 2,
**Characterized in that** at least one joint (56, 58) located in the body separation region (67) is arranged in a height extension region (68) extending along the buoyancy axis (A), in which in the reference state there also extend the two buoyancy volume sections (52a, 54a).

4. Valve array (42) according to one of the preceding Claims,
**Characterized in that** a first virtual swivel axis (S1) about which the first float body (52) is articulated in a swiveling manner at the first joint (56) and a second virtual swivel axis (S2) about which the second float body (54) is articulated in a swiveling manner at the second joint (58) lie in a common virtual extension plane (70), where in the reference state the virtual extension plane (70) preferably intersects a buoyancy volume section (52a, 54a) of the first (52) and of the second float body (54).

5. Valve array (42) according to Claim 4,
**Characterized in that** the virtual extension plane (70) is oriented orthogonally to the buoyancy axis (A).

6. Valve array (42) according to one of the preceding Claims,
**Characterized in that** the valve module (43) comprises a valve housing (60) at which the duct (40) is configured, where each joint (56, 58) comprises a float body-side joint section (56a, 58a) and a bearing-side joint section interacting with the float body-side joint section (56a, 58a), where the bearing-side joint section of at least one joint (56, 58), preferably of both joints (56, 58), is configured at the valve housing (60).

7. Valve array (42) according to Claim 6,
**Characterized in that** the valve housing (60) is configured in one or two parts.

8. Valve array (42) according to one of the preceding Claims, by reference to Claim 4,
**Characterized in that** the first buoyancy volume section (52a) lies closer to the second swivel axis (S2) than to the first swivel axis (S1) and/or that the second buoyancy volume section (54a) lies closer to the first swivel axis (S1) than to the second swivel axis S2).

9. Valve array (42) according to Claim 8,
**Characterized in that** a coupling of the first float body (52) with the valve body formation (50) lies in a region between the first (S1) and the second swivel axis (S2) and/or that a coupling of the second float body (54) with the valve body formation (50) lies in a region between the first (S1) and the second swivel axis (S2).

10. Valve array (42) according to one of the preceding Claims,
**Characterized in that** the first (52) and the second float body (54) are configured identically, where preferably the first (52) and the second float body (54) are arranged twisted relative to one another about a transfer axis (Ub) parallel to the buoyancy axis (A).

11. Valve array (42) according to one of the preceding Claims,
**Characterized in that** the valve seat formation (48) comprises a first valve seat (48a) and a second valve seat (48b) with a separation from the former, where both valve seats (48a, 48b) are penetrated through by the duct (40), and that the valve body formation (50) comprises a first valve body (50a) and a second valve body (50b) moveable relative to it, where the first valve body (50a) is coupled with the first float body (52) for common movement and can be brought into physical abutment against the first valve seat (48a), and where the second valve body (50b) is coupled with the second float body (54) for common movement and can be brought into physical abutment against the second valve seat (48b).

12. Humidification device (16) for a respiratory device (10), comprising a container (20) with a filling volume (24), where the container (20) exhibits an inlet aperture (46) through which respiratory gas be introduced into the filling volume (24), and exhibits an outlet aperture (44) through which respiratory gas can be channeled out of the filling volume (24), where the humidification device (16) exhibits a valve array (42) according to one of the preceding Claims, where the duct (40) of the valve array (42) is a supply duct for introducing fluid into the container (20).

13. Humidification device (16) according to Claim 12,
**Characterized in that** the container (20) exhibits a container bottom (32b) and a side-wall arrangement (32a) sticking out from the container bottom (32b), where for at least one, preferably for both of the float bodies (52, 54) it is the case that the separation of its buoyancy volume section (52a, 54a) from the section of the side-wall arrangement (32b) located next to it is smaller than the separation (a) from the buoyancy volume section (52a, 54a) of the respective other float body (52, 54).

14. Humidification device (16) according to Claim 12 or 13,
**Characterized in that** the volume taken up by the two float bodies (52, 54) comes to no more than 20%, preferably no more than 15% of the filling volume (24) of the container (20).

15. Humidification device (16) according to one of the Claims 12 to 14, **Characterized in that** in normal use with a buoyancy axis (A) oriented in parallel to the gravitational direction (g), the first (52) and the second float body (54) are configured and arranged in such a way that when using demineralized water at a temperature of 20 °C as a reference fluid for filling the container (20), the filling quantity which is needed for the first float body (52) to reach its buoyancy position differs from the filling quantity which is needed for the second float body (54) to reach its buoyancy position by no more than 10%, preferably by no more than 5%, based on the larger of the two filling quantities.

## Revendications

1. Agencement de soupape à flotteur (42), comprenant un ensemble de soupape (43) comportant un canal (40), une formation de siège de soupape (48) traversée par le canal et une formation de corps de soupape (50), la formation de corps de soupape (50) pouvant être déplacée par rapport à la formation de siège de soupape (48) entre une position de fermeture, dans laquelle le canal (40) est fermé par contact physique de la formation de corps de soupape (50) avec la formation de siège de soupape (48), et une position de passage, dans laquelle la formation de corps de soupape (50) est disposée à distance de la formation de siège de soupape (48), de sorte que le canal (40) peut être traversé par un écoulement, l'agencement de soupape (42) comprenant en outre : un premier corps de flotteur (52) comportant une première partie de volume de flottabilité (52a) et un deuxième corps de flotteur (54) comportant une deuxième partie de volume de flottabilité (54a), le premier corps de flotteur (52) étant articulé de manière pivotante sur une première articulation (56) et le deuxième corps de flotteur (54) étant articulé de manière pivotante sur une deuxième articulation (58), de sorte que chaque corps de flotteur (52, 54) est mobile, en fonctionnement normal, le long d'un axe de flottabilité (A) parallèle à la direction d'action de la gravité (g) entre une position abaissée et une position de flottaison, le premier (52) et le deuxième corps de flotteur (54) étant chacun couplés à la formation de corps de soupape (50) de telle sorte que la formation de corps de soupape (50) se trouve alors en position de fermeture lorsqu'au moins l'un des corps de flotteur (52, 54) se trouve en position de flottaison, chaque corps de flotteur (52, 54) s'élevant alors en position de flottaison et fermant le canal (40) lorsqu'une quantité suffisante de liquide s'est accumulée dans sa zone d'agencement, et se trouvant en position de passage lorsque les deux corps de flotteur (52, 54) se trouvent en position abaissée,
**caractérisé en ce que**, en considérant les deux corps de flotteur (52, 54) dans leur position abaissée respective comme état de référence, les parties de volume de flottabilité respectives (52a, 54a) des deux corps de flotteur (52, 54) sont agencés à une distance (a) l'une de l'autre, orthogonale à l'axe de flottabilité (A), au moins une articulation (56, 58) étant située dans une zone de distance des corps (67) entre les deux parties de volume de flottabilité (52a, 54a) ou/et dans laquelle, dans une zone de distance d'articulation entre les deux articulations (56, 58), au moins une partie de volume de flottabilité (52a, 54a) est située.

2. Agencement de soupape (42) selon la revendication 1,
**caractérisé en ce que**, à l'état de référence, les deux articulations (56, 58) sont situées dans la zone de distance des corps (67).

3. Agencement de soupape (42) selon la revendication 1 ou 2,
**caractérisé en ce qu'**au moins une articulation (56, 58) située dans la zone de distance des corps (67) est agencée dans une zone d'étendue en hauteur (68) s'étendant le long de l'axe de flottabilité (A), dans laquelle s'étendent également, à l'état de référence, les deux parties de volume de flottabilité (52a, 54a).

4. Agencement de soupape (42) selon l'une des revendications précédentes, **caractérisé en ce qu'**un premier axe de pivotement virtuel (S1), autour duquel le premier corps de flotteur (52) est articulé de manière pivotante sur la première articulation (56), et un deuxième axe de pivotement virtuel (S2), autour duquel le deuxième corps de flotteur (54) est articulé de manière pivotante sur la deuxième articulation (58), sont situés dans un plan d'extension virtuel commun (70), le plan d'extension virtuel (70) coupant de préférence une partie du volume de flottabilité (52a, 54a) du premier (52) et du deuxième (54) corps de flotteur dans l'état de référence.

5. Agencement de soupape (42) selon la revendication 4, **caractérisé en ce que** le plan d'extension virtuel (70) est orienté orthogonalement à l'axe de flottabilité (A).

6. Agencement de soupape (42) selon l'une des revendications précédentes, **caractérisé en ce que** l'ensemble de soupape (43) comprend un boîtier de soupape (60) auquel le canal (40) est formé, chaque articulation (56, 58) comprenant une partie d'articulation côté corps de flotteur (56a, 58a) et une partie d'articulation côté palier coopérant avec la partie d'articulation côté corps de flotteur (56a, 58a), la partie d'articulation côté palier d'au moins une articulation (56, 58), de préférence des deux articulations (56, 58), étant formée au boîtier de soupape (60).

7. Agencement de soupape (42) selon la revendication 6,
**caractérisé en ce que** le boîtier de soupape (60) est réalisé en une seule pièce ou en deux pièces.

8. Agencement de soupape (42) selon l'une des revendications précédentes, y compris la revendication 4,
**caractérisé en ce que** la première partie de volume de flottabilité (52a) est située plus près du deuxième axe de pivotement (S2) que du premier axe de pivotement (S1) et/ou **en ce que** la deuxième partie de volume de flottabilité (54a) est située plus près du premier axe de pivotement (S1) que du deuxième axe de pivotement (S2).

9. Agencement de soupape (42) selon la revendication 8,
**caractérisé en ce qu'**un couplage du premier corps de flotteur (52) avec la formation de corps de soupape (50) est situé dans une zone entre le premier (S1) et le deuxième axe de pivotement (S2) ou/et **en ce qu'**un couplage du deuxième corps de flotteur (54) avec la formation de corps de soupape (50) est situé dans une zone entre le premier (S1) et le deuxième axe de pivotement (S2).

10. Agencement de soupape (42) selon l'une des revendications précédentes, **caractérisé en ce que** le premier (52) et le deuxième corps de flotteur (54) sont de conception identique, le premier (52) et le deuxième corps de flotteur (54) étant de préférence agencés de manière à être tournés l'un par rapport à l'autre autour d'un axe de transfert (Ub) parallèle à l'axe de flottabilité (A).

11. Agencement de soupape (42) selon l'une des revendications précédentes, **caractérisé en ce que** la formation de siège de soupape (48) comprend un premier siège de soupape (48a) et, à distance de celui-ci, un deuxième siège de soupape (48b), les deux sièges de soupape (48a, 48b) sont traversés par le canal (40), et **en ce que** la formation de corps de soupape (50) comprend un premier corps de soupape (50a) et un deuxième corps de soupape (50b) mobile par rapport à celui-ci, le premier corps de soupape (50a) étant couplé au premier corps de flotteur (52) pour un mouvement conjoint et pouvant être mis en contact physique avec le premier siège de soupape (48a) et le deuxième corps de soupape (50b) étant couplé au deuxième corps de flotteur (54) pour un mouvement conjoint et pouvant être mis en contact physique avec le deuxième siège de soupape (48b).

12. Dispositif d'humidification (16) pour un dispositif de ventilation (10), comprenant un réservoir (20) avec un volume de remplissage (24), le réservoir (20) présentant une ouverture d'entrée (46) à travers laquelle du gaz de ventilation peut être introduit dans le volume de remplissage (24), et une ouverture de sortie (44) à travers laquelle du gaz de ventilation peut être évacué du volume de remplissage (24), le dispositif d'humidification (16) comportant un agencement de soupapes (42) selon l'une des revendications précédentes, le canal (40) de l'agencement de soupapes (42) étant un canal d'alimentation destiné à introduire du liquide dans le réservoir (20).

13. Dispositif d'humidification (16) selon la revendication 12,
**caractérisé en ce que** le réservoir (20) comporte un fond de réservoir (32b) et un agencement de parois latérales (32a) s'étendant à partir du fond de réservoir (32b), au moins un des corps de flotteur (52, 54), de préférence les deux, présentant la particularité que la distance de sa partie de volume de flottabilité (52a, 54a) par rapport à la partie de l'ensemble de parois latérales (32b) qui lui est la plus proche est inférieure à la distance (a) par rapport à la partie de volume de flottabilité (52a, 54a) de l'autre corps de flotteur (52, 54).

14. Dispositif d'humidification (16) selon la revendication 12 ou 13, **caractérisé en ce que** le volume occupé par les deux corps de flotteur (52, 54) ne dépasse pas 20 %, de préférence 15 %, du volume de remplissage (24) du récipient (20).

15. Dispositif d'humidification (16) selon l'une des revendications 12 à 14, **caractérisé en ce que**, lors d'une utilisation conforme à l'usage prévu avec un axe de flottabilité (A) orienté parallèlement à la direction d'action de la gravité (g), le premier (52) et le deuxième corps de flotteur (54) sont conçus et agencés de telle sorte que, lors de l'utilisation d'eau déminéralisée à une température de 20 °C comme liquide de référence pour le remplissage du récipient (20), la quantité de remplissage nécessaire pour que le premier corps de flotteur (52) atteinte sa position de flottaison ne diffère pas de plus de 10 %, de préférence de plus de 5 %, de la quantité de remplissage nécessaire pour que le deuxième corps de flotteur (54) atteinte sa position de flottaison, rapporté à la plus grande des deux quantités de remplissage.
